# EUROPÄISCHE PATENTANMELDUNG

(11) **EP 0 251 012 A2**
(43) Veröffentlichungstag der Anmeldung: **07.01.1988**
(21) Anmeldenummer: 87108591.6
(22) Anmeldetag: 15.06.1987
(51) Int. Cl.: C07D 235/10, C07D 491/04, A01N 43/52, A01N 43/90

(54) **N-Sulfenylierte 2-Trifluormethylbenzimidazole**

(30) Priorität: 25.06.1986 DE 3621265
(71) Anmelder: BAYER AG, 51368 Leverkusen (DE)
(72) Erfinder: Heywang, Gerhard, Dr., D-5060 Bergisch Gladbach 2 (DE); Baasner, Bernd, Dr., D-5090 Leverkusen 3 (DE); Hänssler, Gerd, Dr., D-5090 Leverkusen 3 (DE); Paulus, Wilfried, Dr., D-4150 Krefeld (DE); Santel, Hans-Joachim, Dr., D-5090 Leverkusen 1 (DE); Schmidt, Robert R., Dr., D-5060 Bergisch Gladbach 2 (DE); Schmitt, Hans-Georg, Dr., D-4150 Krefeld 1 (DE)

(57) **Zusammenfassung**

Die Erfindung betrifft neue N-Sulfenylierte 2-Trifluormethyl-benzimidazole der allgemeinen Formel (I) 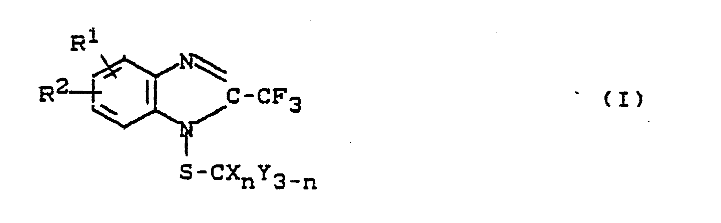 in welcher
X und Y unabhängig voneinander für Halogen stehen,
n für 0, 1, 2 oder 3 steht und
R¹ und R² gleich oder verschieden sind und für Wasserstoff, Halogen, jeweils gegebenenfalls durch Halogen substituiertes Alkyl, Alkoxy oder Alkylthio stehen oder gemeinsam für einen gegebenenfalls durch Halogen substituierten und gegebenenfalls durch Heteroatome unterbrochenen ankondensierten Ring stehen,
ein Verfahren zu ihrer Herstellung sowie ihre Verwendung als Herbizide und Mikrobizide.

## Beschreibung

Die Erfindung betrifft neue N-Sulfenylierte 2-Trifluormethyl-benzimidazole, ein Verfahren zu ihrer Herstellung sowie ihre Verwendung als Herbizide und Mikrobizide.

Es ist bereits bekannt, daß bestimmte N-Sulfenylierte Indazole fungizide und mikrobizide Eigenschaften aufweisen (vgl. US-P-3 867 540). Die Wirkung dieser Verbindungen ist jedoch in bestimmten Indikationsgebieten, unter bestimmten Bedingungen, wie z.B. bei niedrigen Aufwandmengen und -konzentrationen, nicht immer befriedigend.

Es wurden nun neue N-Sulfenylierte 2-Trifluoromethylbenzimidazole der allgemeinen Formel (I) gefunden, 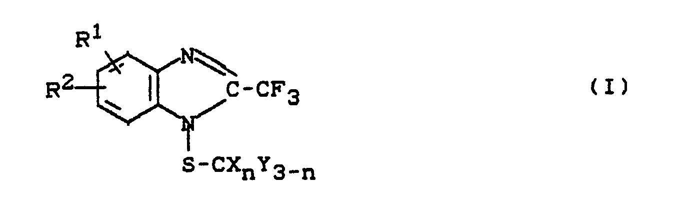 in welcher
X und Y unabhängig voneinander für Halogen stehen,
n für 0, 1, 2 oder 3 steht und
R¹ und R² gleich oder verschieden sind und für Wasserstoff, Halogen, jeweils gegebenenfalls durch Halogen substituiertes Alkyl, Alkoxy oder Alkylthio, für Nitro oder Alkylsulfonyl stehen oder gemeinsam für einen gegebenenfalls durch Halogen substituierten und gegebenenfalls durch Heteroatome unterbrochenen ankondensierten Ring stehen.

Weiterhin wurde gefunden, daß man die neuen N-Sulfenylierten 2-Trifluormethyl-benzimidazole der Formel (I), 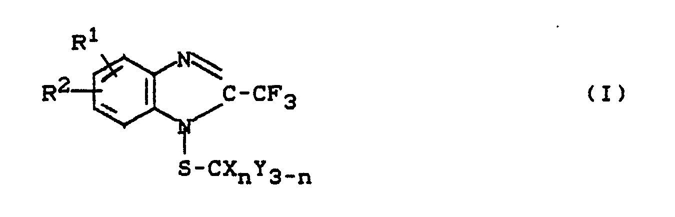 in welcher
X und Y unabhängig voneinander für Halogen stehen,
n für 0, 1, 2 oder 3 steht und
R¹ und R² gleich oder verschieden sind und für Wasserstoff, Halogen, jeweils gegebenenfalls durch Halogen substituiertes Alkyl, Alkoxy oder Alkylthio, für Nitro oder Alkylsulfonyl stehen oder gemeinsam für einen gegebenenfalls durch Halogen substituierten und gegebenenfalls durch Heteroatome unterbrochenen ankondensierten Ring stehen,
erhält, wenn man 2-Trifluoromethyl-benzimidazole der Formel (II) 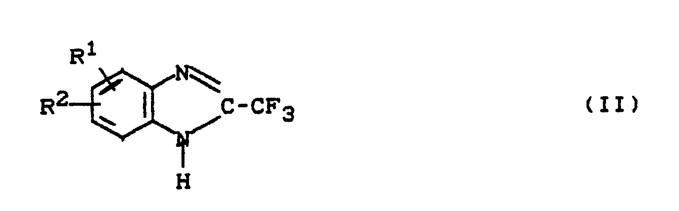 in welcher
R¹ und R² gleich oder verschieden sind und die oben angegebene Bedeutung haben,
mit Sulfensäurehalogeniden der Formel (III)
Z-S-CXₙY₃₋ₙ (III)
in welcher
X, Y und n die oben angegebene Bedeutung haben und
Z für Halogen, bevorzugt für Chlor oder Brom, steht,
in Gegenwart eines Verdünnungsmittels und in Gegenwart eines Säurebindemittels umsetzt.

Schließlich wurde gefunden, daß die neuen N-Sulfenylierten 2-Trifluormethyl-benzimidazole der Formel (I) starke herbizide und mikrobizide Eigenschaften aufweisen.

Überraschenderweise zeigen die erfindungsgemäßen Wirkstoffe der Formel (I) eine höhere herbizide und antimikrobielle Wirksamkeit als die aus dem Stand der Technik bekannten Verbindungen gleicher Wirkungsart.

Im Rahmen der Substituentendefinition steht Halogen für Fluor, Chlor, Brom und Iod, bevorzugt für Fluor, Chlor und Brom, besonders bevorzugt für Fluor und Chlor.

Alkyl steht vorzugsweise für eine Kohlenstoffkette mit 1 bis 6, besonders bevorzugt 1 bis 4 und ganz besonders bevorzugt mit 1 oder 2 Kohlenstoffatomen, wie beispielsweise Methyl, Ethyl, n-Propyl, iso-Propyl, n-Butyl, iso-Butyl, sek.-Butyl, tert.-Butyl, Pentyl und Hexyl.

Alkoxy steht vorzugsweise für Alkoxy mit einer Kohlenstoffkette aus 1 bis 4, besonders bevorzugt 1 bis 3 und ganz besonders bevorzugt aus 1 oder 2 Kohlenstoffatomen, wie beispielsweise Methoxy, Ethoxy, n- und i-Propoxy.

Alkylthio steht vorzugsweise für Alkylthio mit einer Kohlenstoffkette mit 1 bis 4, besonders bevorzugt aus 1 bis 3 Kohlenstoffatomen und ganz besonders bevorzugt aus 1 Kohlenstoffatom.

Alkylsulfonyl steht vorzugsweise für Alkylsulfonyl mit einer Kohlenstoffkette mit 1 bis 4, besonders bevorzugt 1 oder 2 Kohlenstoffatomen.

R¹ und R² stehen gemeinsam auch für einen ankondensierten, bevorzugt 5- oder 6-gliedrigen Ring, der durch Sauerstoff, Schwefel und/oder Stickstoff, bevorzugt durch 1 oder 2 Sauerstoff- und/oder Schwefelatome unterbrochen ist und welcher ein- oder mehrfach durch Halogen, bevorzugt Fluor, Chlor und Brom, insbesondere durch Fluor und Chlor substituiert sein kann. R¹ und R² stehen insbesondere für die Reste -O-CHF-CF₂-O-, -O-CF₂-CFCl-O-, -O-CF₂-CF₂-O-, -O-CF₂-O- und -O-CF₂-O-CF₂-, n steht im allgemeinen für 0, 1, 2 oder 3, bevorzugt für 1, 2 oder 3, besonders bevorzugt für 2 oder 3 und insbesondere für 2.

Bevorzugt sind N-sulfenylierte 2-Trifluormethylbenzimidazole der Formel (I), in welcher
X und Y unabhängig voneinander für Fluor, Chlor und Brom stehen,
n für 0, 1, 2 oder 3 steht, und
R¹ und R² gleich oder verschieden sind und für Wasserstoff, Fluor, Chlor, Brom, jeweils gegebenenfalls ein- oder mehrfach durch Fluor, Chlor und/oder Brom substituiertes Alkyl mit 1 bis 6 Kohlenstoffatomen, Alkoxy und Alkylthio mit jeweils 1 bis 4 Kohlenstoffatomen, für Nitro oder Alkylsulfonyl mit 1 bis 4 Kohlenstoffatomen stehen oder R¹ und R² gemeinsam für einen 5- oder 6-gliedrigen, ankondensierten Ring stehen, welcher durch Sauerstoff, Stickstoff und/oder Schwefel unterbrochen und ein- oder mehrfach durch Fluor, Chlor und/oder Brom substituiert sein kann.

Besonders bevorzugt sind diejenigen Verbindungen der Formel (I), in welcher
X und Y unabhängig voneinander für Fluor, Chlor und Brom stehen,
n für 2 oder 3 steht und
R¹ und R² gleich oder verschieden sind und für Wasserstoff, Fluor, Chlor, Brom, jeweils gegebenenfalls ein- bis fünffach durch Fluor, Chlor und/oder Brom substituiertes Alkyl mit 1 bis 4 Kohlenstoffatomen, Alkoxy oder Alkylthio mit jeweils 1 bis 3 Kohlenstoffatomen, für Nitro oder für Alkylsulfonyl mit 1 oder 2 Kohlenstoffatomen stehen oder R¹ und R² gemeinsam für einen 5- oder 6-gliedrigen, ankondensierten Ring stehen, welcher durch 1 oder 2 Sauerstoff- und/oder Schwefelatome unterbrochen und ein- bis vierfach durch Fluor, Chlor und/oder Brom substituiert sein kann.

Ganz besonders bevorzugt sind diejenigen N-sulfenylierten 2-Trifluormethyl-benzimidazole der Formel (I), in welcher
X und Y unabhängig voneinander für Fluor oder Chlor stehen,
n für 2 oder 3 steht und
R¹ und R² gleich oder verschieden sind und für Wasserstoff, Fluor, Chlor, Brom, Methyl, Fluormethyl, Difluormethyl, Trifluormethyl, Chlormethyl, Dichlormethyl, Trichlormethyl, Chlordifluormethyl, Difluorethyl, Methoxy, Fluormethoxy, Dichlormethoxy, Trifluormethoxy, Chlordifluormethoxy, Dichlorfluormethoxy, 1,1,2,2-Tetrafluorethoxy, 2-Chlor-1,1,2-trifluorethoxy, 2,2,2-Trifluorethoxy, 2,2-Dichlor-1,1,2-trifluorethoxy, 2-Chlor-1,1,2,2-tetrafluorethoxy, 2-Chlor-2,2-difluorethoxy, 2,2-Dichlor-2-fluorethoxy, 2,2,2-Trichlorethoxy, 1,1,2,3,3,3-Hexafluorpropoxy, Methylthio, Chlordifluormethylthio, Difluormethylthio, Trifluormethylthio, Nitro, Methylsulfonyl, Ethylsulfonyl, 5,6-Dioxymethylen, 5,6-Dioxydifluormethylen, 5,6-Dioxy-chlorfluormethylen, 5,6-Dioxyethylen, 5,6-Dioxy-difluorethylen, 5,6-Dioxytrifluorethylen, 5,6-Dioxytetrafluorethylen, 5,6-Dioxy-chlortrifluorethylen, 5,6-Dioxy-dichlordifluorethylen, 5,6-Dioxy-chlordifluorethylen, 5,6-Dioxychlorfluorethylen oder 5,6-Dioxy-dichlorfluorethylen oder 6,7-Oxydifluormethylenoxydifluormethylen stehen.

Beispielhaft seien die folgenden N-sulfenylierten 2-Trifluormethyl-benzimidazole der Formel (I) namentlich aufgeführt. Hierbei sind bezüglich des Sulfenylrestes auch Stellungsisomere denkbar:
1-Dichlorfluormethylsulfenyl-4-trifluormethoxy-2-trifluormethyl-benzimidazol,
1-Dichlorfluormethylsulfenyl-5-trifluormethoxy-2-trifluormethyl-benzimidazol,
1-Dichlorfluormethylsulfenyl-6-trifluormethoxy-2-trifluormethyl-benzimidazol,
1-Dichlorfluormethylsulfenyl-7-trifluormethoxy-2-trifluormethyl-benzimidazol,
1-Dichlorfluormethylsulfenyl-4-trifluormethyl-7-chlor-2-trifluormethyl-benzimidazol,
1-Dichlorfluormethylsulfenyl-5-difluormethoxy-2-trifluormethyl-benzimidazol,
1-Dichlorfluormethylsulfenyl-[2-chlor-1,1,2-trifluorethoxy]-2-trifluormethyl-benzimidazol,
1-Dichlorfluormethylsulfenyl-5-chlordifluormethoxy-2-trifluormethyl-benzimidazol,
1-Dichlorfluormethylsulfenyl-4-chlor-5-trifluormethoxy-2-trifluormethyl-benzimidazol,
1-Dichlorfluormethylsulfenyl-4-methyl-5-trifluormethoxy-2-trifluormethyl-benzimidazol,
1-Dichlorfluormethylsulfenyl-5-brom-6-fluor-2-trifluormethyl-benzimidazol,
1-Dichlorfluormethylsulfenyl-5,7-dimethyl-6-trifluormethyl-2-trifluormethyl-benzimidazol,
1-Dichlorfluormethylsulfenyl-4-trifluormethylthio-2-trifluormethyl-benzimidazol,
1-Dichlorfluormethylsulfenyl-5-trifluormethylthio-2-trifluormethyl-benzimidazol,
1-Dichlorfluormethylsulfenyl-6-trifluormethylthio-2-trifluormethyl-benzimidazol,
1-Dichlorfluormethylsulfenyl-7-trifluormethylthio-2-trifluormethyl-benzimidazol,
1-Dichlorfluormethylsulfenyl-5-dichlorfluormethylthio-2-trifluormethyl-benzimidazol,
1-Dichlorfluormethylsulfenyl-5-difluormethoxy-2-trifluormethyl-benzimidazol,
1-Dichlorfluormethylsulfenyl-5-trifluormethyl-2-trifluormethyl-benzimidazol,
1-Dichlorfluormethylsulfenyl-4-[2,2,2-trifluorethoxy]-2-trifluormethyl-benzimidazol,
1-Dichlorfluormethylsulfenyl-5-[2,2,2-trifluorethoxy]-2-trifluormethyl-benzimidazol,
1-Dichlorfluormethylsulfenyl-4-[1,1,2,2-tetrafluorethoxy]-2-trifluormethyl-benzimidazol,
1-Dichlorfluormethylsulfenyl-5-[1,1,2,2-tetrafluorethoxy]-2-trifluormethyl-benzimidazol,
1-Dichlorfluormethylsulfenyl-5,6-[dioxy-tetrafluorethylen]-2-trifluormethyl-benzimidazol,
1-Dichlorfluormethylsulfenyl-5,6-[dioxy-chlortrifluorethylen]-2-trifluormethyl-benzimidazol,
1-Dichlorfluormethylsulfenyl-6-[2-chlor-1,1,2-trifluorethoxy]-5-methyl-2-trifluormethyl-benzimidazol,
1-Dichlor-fluormethylsulfonyl-5-[2-chlor-1,1,2-trifluorethoxy]-6-methyl-2-trifluormethyl-benzimidazol,
1-Dichlorfluormethylsulfenyl-5,6-bistrifluormethoxy-2-trifluormethyl-benzimidazol.

Verwendet man beispielsweise 5-Trifluormethoxy-2-trifluormethyl-benzimidazol und Dichlorfluormethansulfensäurechlorid als Ausgangsstoffe, so kann der Reaktionsablauf des erfindungsgemäßen Verfahrens durch das folgende Formelschema wiedergegeben werden:

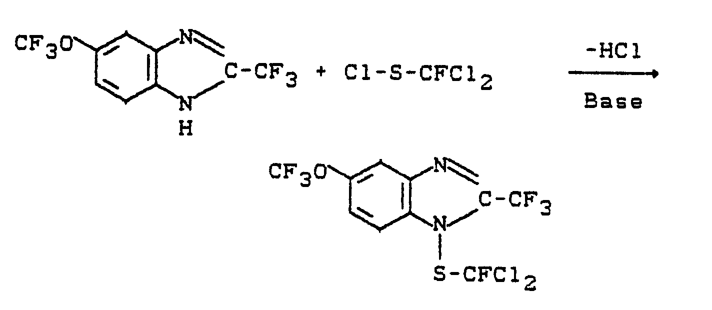

Die zur Durchführung des erfindungsgemäßen Verfahrens als Ausgangsstoffe benötigten 2-Trifluormethyl-benzimidazole sind durch die Formel (II) allgemein definiert.

R¹ und R² haben darin vorzugsweise die Bedeutung, die bereits im Zusammenhang mit der Beschreibung der erfindungsgemäßen Stoffe der Formel (I) vorzugsweise für diese Symbole genannt wurde.

Die 2-Trifluormethyl-benzimidazole der Formel (II) sind teilweise bekannt (vgl. z.B. DE-OS 1 642 334), teilweise sind sie Gegenstand einer parallel eingereichten deutschen Patentanmeldung P 3 621 301 2.

Die Verbindungen der Formel (II) werden nach im Prinzip bekannten Verfahren hergestellt (vgl. z.B. M.A. Phillips, J. Chem. Soc. 1928, 2393-2399 und W.T. Smith, E.C. Steinle, J. Am. Chem. Soc. 75, 1292-1294 (1952)).

Man erhält sie beispielsweise, wenn man o-Phenylendiamine der Formel (IV) 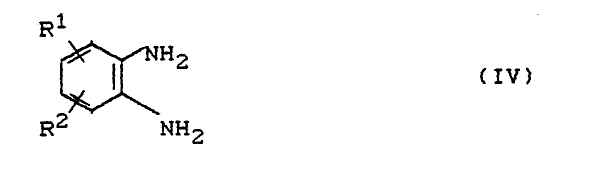 in welcher
R¹ und R² die oben angegebene Bedeutung haben,
mit Trifluoressigsäure in Gegenwart von 4N Salzsäure und gegebenenfalls in Gegenwart eines Verdünnungsmittel, wie beispielsweise Wasser, unter Rückfluß erhitzt oder wenn man die Hydrochloride der o-Phenylendiamine der Formel (IV) in analoger Weise umsetzt.

Die Ausgangsstoffe der Formel (IV) sind teilweise bekannt (vgl. z.B. EP-A- 127.763). Die noch nicht bekannten o-Phenylendiamine der Formel (IV) sind teilweise Gegenstand einer nicht vorveröffentlichen Patentanmeldung DE-P 36 05 977 vom 25. Februar 1986 und teilweise einer parallel eingereichten deutschen Patentanmeldung P 3 621 215 6.

Die neuen und auch die bekannten Verbindungen der allgemeinen Formel (IV) können hergestellt werden (vgl. z.B. L.M. Yagupolskij, et. al., Zh. obsh. Khim 33, 3051-5 (1963); Houben-Weyl, Band X/1, S. 559 (1971) und Band XI/1, S. 472-3 (1957) und Herstellungsbeispiele), indem man beispielsweise Verbindungen der Formel (V) 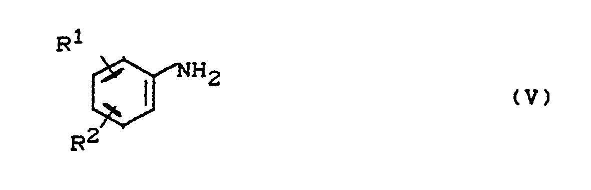 in der
R¹ und R² die oben angegebene Bedeutung haben,
zunächst an der Aminogruppe acyliert, dann in 2-Stellung zur acylierten Aminogruppe mit einem Nitrierungsmittel wie beispielsweise Nitriersäure, gegebenenfalls in Gegenwart eines Verdünnungsmitels wie beispielsweise Eisessig, und gegebenenfalls in Gegenwart eines Katalysators, wie beispielsweise Acetanhydrid bei Temperaturen zwischen -20 und +50°C eine Nitrogruppe einführt, die Nitrogruppe in Gegenwart eines Katalysators, wie beispielsweise Raney-Nickel, und in Gegenwart eines Verdünnungsmittels, wie beispielsweise Methanol, bei Wasserstoffdrucken von 10 bis 100 bar und bei Temperaturen zwischen +20 und +80°C zur Aminogruppe hydriert und dann die Acylgruppe in üblicher Weise, beispielsweise durch Verseifung mit wäßriger oder alkoholischer Base, wieder abspaltet.

Die Verbindungen der Formel (V) sind bekannt und lassen sich nach bekannten Verfahren in analoger Weise herstellen (vgl. z.B. EP 11 179).

Die zur Durchführung des erfindungsgemäßen Verfahrens weiterhin als Ausgangsstoffe benötigten Sulfensäurehalogenide der Formel (III) sind allgemein bekannte Verbindungen der organischen Chemie. In dieser Formel haben X, Y und n vorzugsweise die Bedeutung, die bereits bei der Beschreibung der erfindungsgemäßen Stoffe der Formel (I) vorzugsweise für diese Reste genannt wurden, Z steht bevorzugt für Chlor oder Brom.

Als Lösungs- und/oder Verdünnungsmittel kommen für das erfindungsgemäße Verfahren insbesondere in Frage: aromatische Kohlenwasserstoffe wie Benzol oder Toluol; halogenierte Kohlenwasserstoffe wie Methylenchloride oder Tetrachlorkohlenstoff: Nitrile wie Acetonitril oder Propionitril: Ether, wie Tetrahydrofuran oder Dioxan: sowie Ester wie Essigsäureethylester.

Als Säurebindemittel können für das erfindungsgemäße Verfahren alle üblichen organischen und anorganischen Säurebinder eingesetzt werden. Hierzu gehören vorzugsweise tertiäre Amine wie Triethylamin oder Pyridin: Alkalihydroxide und/oder Alkalicarbonate wie Natrium- und Kaliumhydroxid, sowie Natriumhydrogencarbonat und Kaliumcarbonat.

Die Reaktionstemperaturen können bei der Durchführung des erfindungsgemäßen Verfahrens in einem größeren Bereich variiert werden. Im allgemeinen arbeitet man bei Temperaturen zwischen 0 und 150°C, vorzugsweise zwischen 20 und 100°C.

Zur Durchführung des erfindungsgemäßen Verfahrens setzt man die Indazole der Formel (II) und die Sulfensäurehalogenide der Formel (III) im allgemeinen in äquimolaren Mengen ein. Vorzugsweise setzt man pro Mol Trifluormethylbenzimidazol der Formel (II) etwa 0,8 bis 3, besonders bevorzugt 0,85 bis 1,5 Mol Sulfensäurehalogenid der Formel (III) ein.

Die Isolierung der neuen N-Sulfenylierten 2-Trifluormethylbenzimidazole der allgemeinen Formel (I) erfolgt in allgemein üblicher Art und Weise, durch beispielsweise Versetzen des Reaktionsgemisches mit Wasser, Extraktion mit einem organischen Lösungsmittel, Chromatographie oder Destillation.

Bei den Benzimidazolen der Formel (I), die sich von symmetrischen Diaminen der Formel (II) ableiten, ist es durch geeignete Reaktionsführung möglich, unterschiedlich am Stickstoff sulfenylierte Produkte oder Gemische davon zu erhalten.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich zur Bekämpfung von mono- und dikotylen Unkräutern, im Vor- und Nachauflaufverfahren in mono-und dikotylen Kulturen, wie beispielsweise Baumwolle, Mais und Weizen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchloride, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorgansiche Pigmente, z.B. Eisenoxid, Titanoxid, Ferrocyanblau und organsiche Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten bei der Anwendung als Herbizid oder Fungizid im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,Nʹ-dimethyl-harnstoff zur Unkrautbekämpfung in Getreide: 4-Amino-3-methyl-6-phenyl-1,2,4-triazin-5(4H)-on zur Unkrautbekämpfung in Zuckerrüben und 4-Amino-6-(1,1,-dimethylethyl)-3-methylthio-1,2,4-triazin-5(4H)-on zur Unkrautbekämpfung in Sojabohnen, in Frage.

Auch Mischungen mit Chloressigsäure-N-(methoxymethyl)-2,6-diethylanilid, 2-Ethyl-6-methyl-N-(1-methyl-2-methoxyehtyl)-chloracetanilid, N-Methyl-2-(1,3-benzthiazol-2-yloxy)-acetanilid, N,N-Diisopropyl-S-(2,3,3-trichlorallyl)-thiolcarbamat, N,N-Di-n-propyl-thiocarbamidsäure-S- ethylester, exo-1-Methyl-4-(1-methylethyl)-2-(2-methylphenylmethoxy)-7-oxabicyclo-[2.2.1]-heptan, 1-Methyl-3-phenyl-5-[3-trifluormethyl-phenyl]-4(1H)-pyridinon, Methyl-2-[4,5-dihydro-4-methyl-4-(1-methylethyl)-5-oxo-1H-imidazol-2-yl]-4(5)-methylbenzoat, 2-[1-(Ethoxamino)-butyliden]-5-(2-ethylthiopropyl)-1,3-cyclohexandion, 3-Isopropyl-2,1,3-benzothiadiazin-4-on-2,2-dioxid, 6-Chlor-3-phenyl-pyridazin-4-yl-5-octyl-thiocarbonat, N-Benzthiazolyl-N-methyl-Nʹ-methylharnstoff, N,N-Dimethyl-Nʹ-(3-chlor-4-methylphenyl-harnstoff, 2-Chlor-N-{[(4-methoxy-6-methyl-1,3,5-triazin-2-yl)-amino]-carbonyl}-benzolsulfonamid, N,N-Dimethyl-Nʹ-(4-isopropylphenyl)-harnstoff, N,N-Dimethyl-Nʹ-(3-trifluormethyl-phenyl)-harnstoff, 4-Ethylamino-2-t-butylamino-6-methylthio-s-triazin, 2-Chlor-4,6-bis(ethylamino)-1,3,5-triazin, 2-Chlor-4-ethylamino-6-isopropylamino-1,3,5-triazin, 2-Chlor-4-ethylamino-6-(3-cyanopropylamino)-1,3,5-triazin, 4-Amino-6-tert.-butyl-3-methylthio-4,5-dihydro-1,2,4-triazin-5-on, 2-[4,5-Dichlorpyrid-2-yloxy)-phenoxy]-propionsäure-trimethylsilymethylester, 2-{4-[(3-Chlor-5-trifluormethyl-2-pyridyl}-oxy]-phenoxy}-propansäure, 2-[4-(2,4-Dichlorphenoxy)-phenoxy]-propionsäuremethylester, 2,4-Dichlorphenoxyessigsäure, 2,4-Dichlorphenoxy-propionsäure, (2-Methyl-4-chlorphenoxy)-essigsäure, (4-Chlor-2-methyl-phenoxy)-propionsäure, Methyl-5-(2,4-dichlorphenoxy)-2-nitrobenzoat, 3,5-Diiod-4-hydroxybenzodinitril, 3,5-Dibrom-4-hydroxybenzonitril, N-(1-Ethoxypropyl)-3,4-dimethyl-2,6-dinitroanilin kommen in Frage. Einige Mischungen zeigen überraschenderweise auch synergistische Wirkung.

Auch eine Mischung mit anderen bekannten Wirkstoffen, wie Fungiziden, Insektiziden, Arkariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizid in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die N-sulfenylierten 2-Trifluormethyl-benzimidazole der Formel (I), insbesondere die N-Dichlorfluormethylthio-2-trifluormethyl-benzimidazole, weisen auch eine starke mikrobizide Wirkung auf und können zur Bekämpfung von unerwünschten Mikroorganismen eingesetzt werden. Die Wirkstoffe sind für den Gebrauch als Pflanzenschutzmittel, insbesondere als Fungizide, geeignet oder im Materialschutz zum Schutz technischer Materialien einsetzbar.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis:
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Bei der Behandlung von Pflanzenteilen können die Wirkstoffkonzentrationen bei der Anwendung als Fungizid in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Darüber hinaus weisen die erfindungsgemäßen Wirkstoffe der Formel (I) eine gute Wirksamkeit bei der Bekämpfung von Bodeninsekten, Nematoden und Hygieneschädlinge auf.

Technische Materialien sind erfindungsgemäß nicht lebende Materialien, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die duch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungs gemäßen Wirkstoffe gegen Pilze, insbesondere Schimmelpilze, holzverfärbende und holzzerstörende Pilze (Basidiomyceten), sowie gegen Bakterien, Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Schlerophoma, wie Sclerophoma pityophila,
Trichoderma, wie Trichoderma viride,
Escherichia, wie Escherichia coli,
Pseudomonas, wie Pseudomonas aeriginosa,
Staphylococcus, wie Staphylococcus aureus.

Je nach Anwendungsgebiet kann ein erfindungsgemäßer Wirkstoff in die üblichen Formulierungen überführt werden, wie Lösungen, Emulsionen, Suspensionen, Pulver, Pasten und Granulate.

Diese können in an sich bekannter Weise hergestellt werden, z.B. durch Vermischen der Wirkstoffe mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für die Wirkstoffe können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten bei der Anwendung zum Schutz technischer Materialien die Wirkstoffe im allgemeinen in einer Menge von 1 bis 95%, bevorzugt von 10 bis 75%.

Die Anwendungskonzentrationen der erfindungsgemäßen Wirkstoffe richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Die erfindungsgemäßen Wirkstoffe können auch in Mischung mit anderen bekannten Wirkstoffen vorliegen. Beispielsweise seien die folgenden Wirkstoffe genannt: Benzylalkoholmono(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanidomethylthiobenzthiazol, Organo-Zinnverbindungen, Methylenbisthiocyanat, Phenolderivate, wie 2-Phenylphenol, (2,2ʹ-Dihydroxy-5,5ʹ-dichlor)diphenylmethan und 3-Methyl-4-chlor-phenol.

Die Herstellung und die Verwendung der erfindungsgemäßen Wirkstoffe geht aus den nachfolgenden Beispielen hervor:

### Herstellungsbeispiele

### Beispiel 1

1-Dichlorfluormethylsulfenyl-2,5-bis(trifluormethyl)-benzimidazol 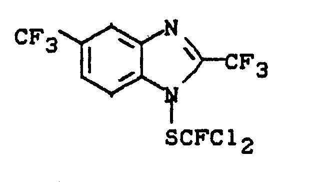 Zu 25,4 g (0,1 Mol) 2,6-Bis(trifluormethyl)-benzimidazol und 10,1 g (0,1 Mol) Triethylamin in 120 ml Dichlormethan werden zwischen 20°C und 30°C innerhalb von etwa einer Stunde 16,9 g (0,1 Mol) Dichlorfluormethylsulfenylchlorid, gelöst in 120 ml Dichlormethan, zugetropft. Es wird 2 h bei gleicher Temperatur nachgerührt, das ausgefallene Triethylamin-Hydrochlorid abgesaugt und mit Dichlormethan nachgewaschen. Das mit der Waschphase vereinigte Filtrat wird zweimal mit Wasser gewaschen. Nach dem Trocknen (MgSO₄) werden die flüchtigen Bestandteile im Wasserstrahlvakuum abdestilliert. Der ölige Rückstand wird über eine kurze Kieselgelsäule chromatografisch filtriert (Fließmittel: Toluol).
Ausbeute: 32,4 g (83,8 % der Theorie).
Siedepunkt: 103-105°C/0,2 mbar

In der nachfolgenden Tabelle sind die auf analoge Weise hergestellten Verbindungen der Formel (I) aufgeführt. Die Verbindungen wurden zur Reinigung umkristallisiert, destilliert oder durch Chromatografie an Kieselgel mit Dichlormethan oder Toluol als Laufmittel gereinigt.

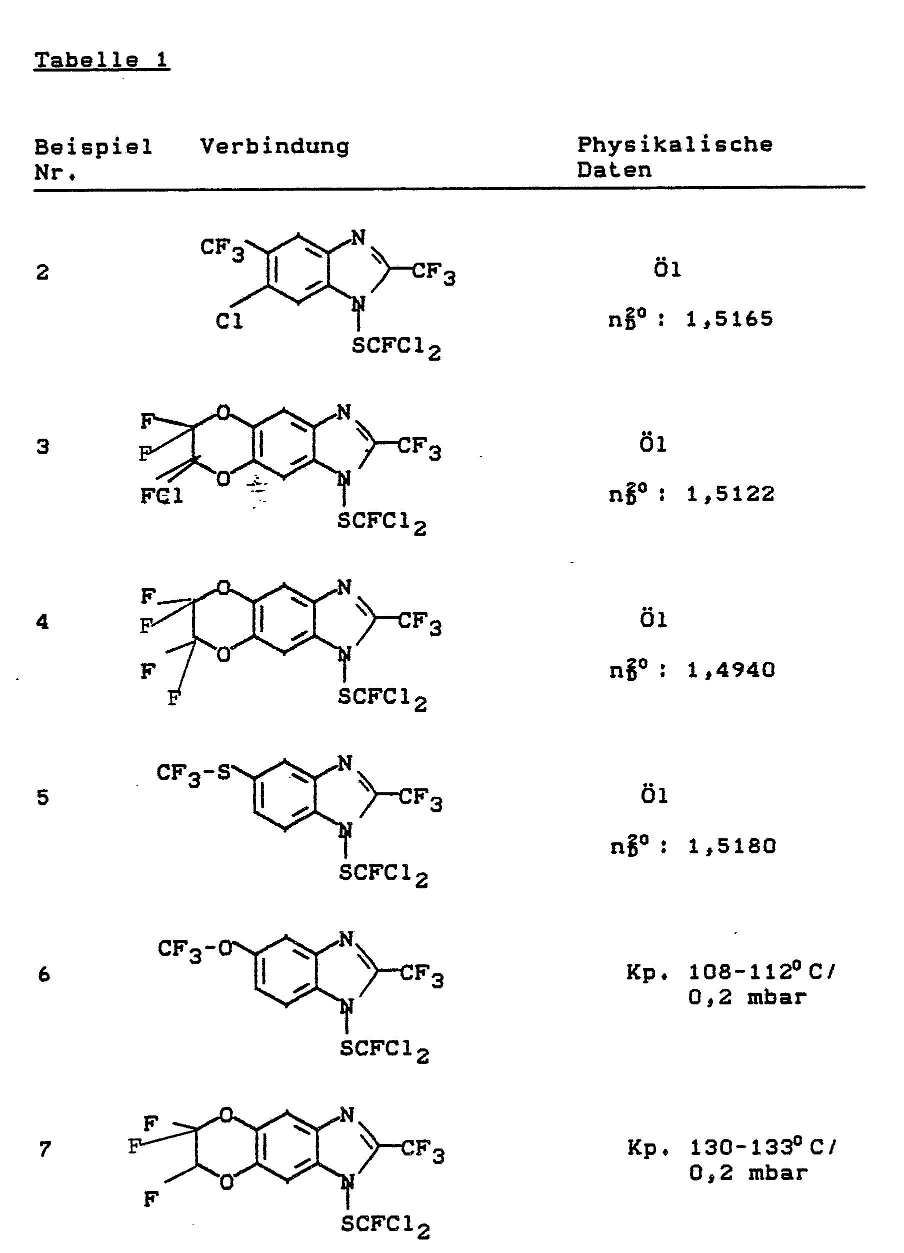

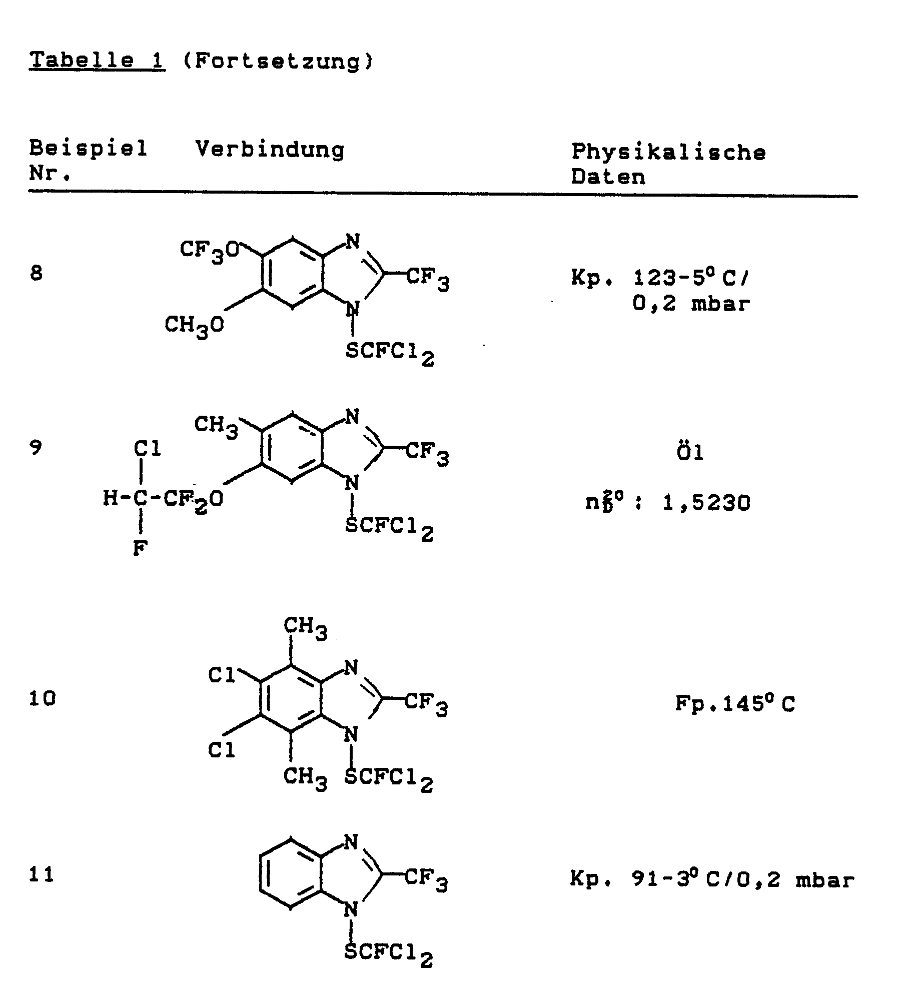

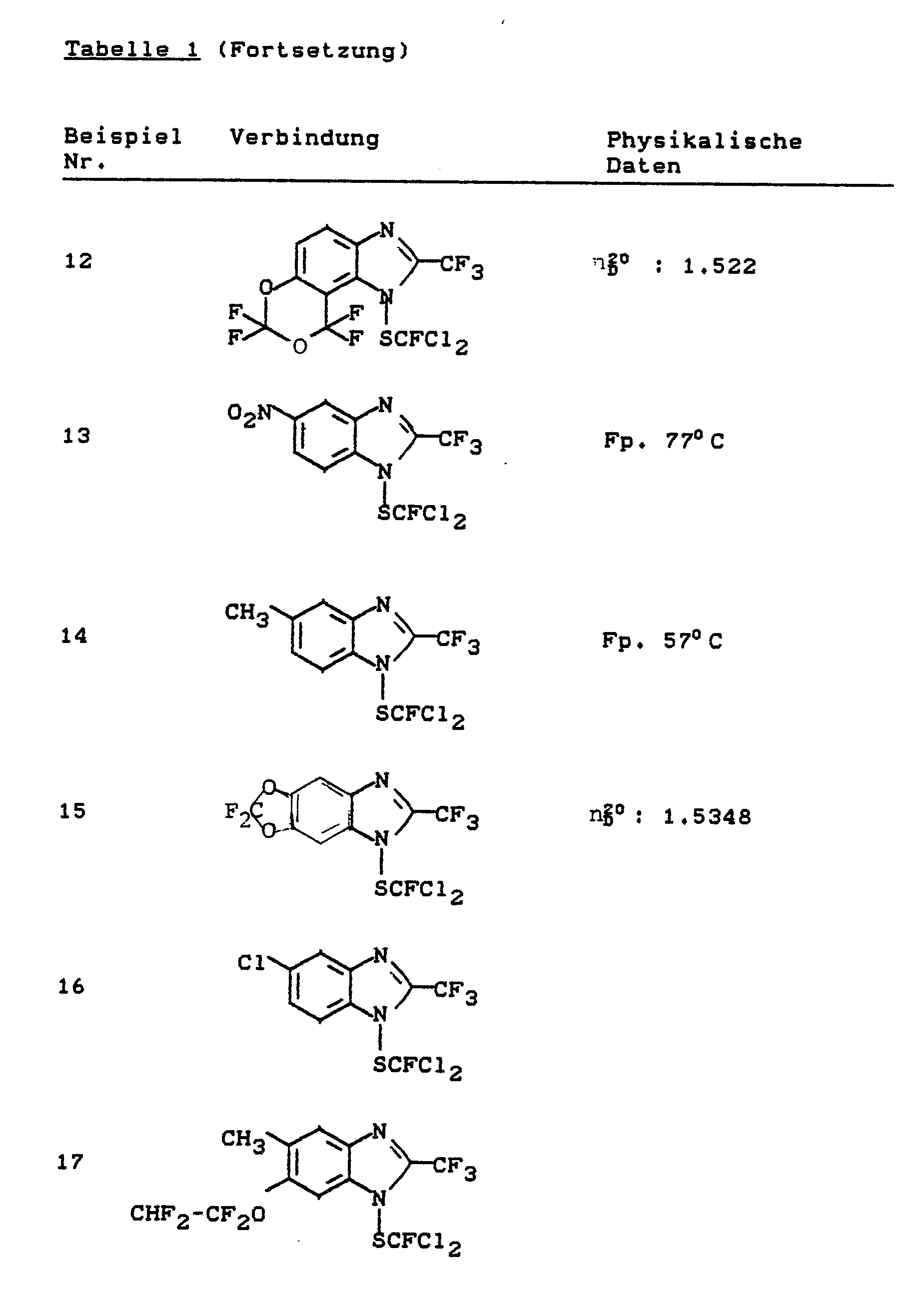

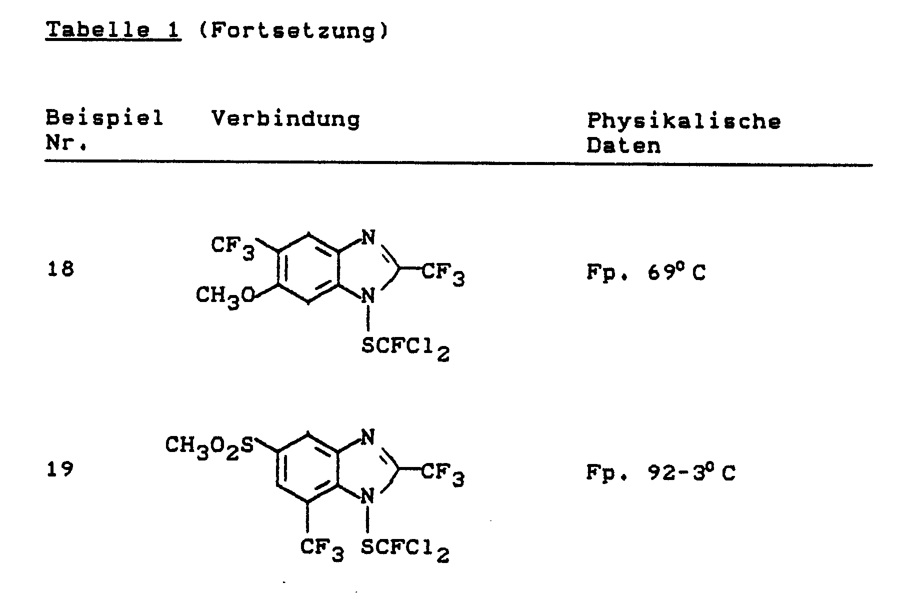

### Anwendungsbeispiele

### Beispiel A

### Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen z.B. die Verbindungen gemäß den Herstellungsbeispielen 1 und 2 neben einer sehr guten herbiziden Wirksamkeit gegen dikotyle Unkräuter, wie bei spielsweise Chenopodium, Datura, Matricaria, Portulak und Stellaria und monokotyle Unkräuter, wie beispielsweise Cynodon eine sehr gute Nutzpflanzenverträglichkeit, insbesondere in Baumwolle und Weizen.

### Beispiel B

### Post-emergence-Test

Lösungsmittel: 5 Gewichtsteile Aceton
Emulgator: 1 Gewichtsteil Alkylarylpolyglyolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen z.B. die Verbindungen gemäß der Herstellungsbeispiele 1, 2 und 3 neben einer sehr guten herbiziden Wirkung gegen dikotyle Unkräuter, wie beispiels weise Chenopodium, Sinapis, Solanum und gegen monokotyle Unkräuter, wie beispielsweise Echinochloa und Poa, auch eine sehr gute Nutzpflanzenverträglichkeit, insbesondere in Mais und Baumwolle.

### Beispiel C

### Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermsicht man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß der Herstellungsbeispiele 2 und 6.

### Beispiel D

Zum Nachweis der Wirksamkeit gegen Pilze werden die minimalen Hemm-Konzentrationen (MHK) von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 5000 mg/l versetzt. Nach Erstarren des Agars erfolgt Kontamination mit Reinkulturen der in der Tabelle aufgeführten Testorganismen. Nach 2-wöchiger Lagerung bei 28°C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt. Gute Wirkungen zeigen z.B. die Verbindungen gemäß den Herstellungsbeispielen 1, 2, 3, 5, 6, 7 und 8 an den Testorganismen:

Alternaria tenuis, Asperigillus niger, Aureobasidium pullulans, Chaetomium globosum, Cladosporium cladosporioides, Lentinus tigrinus, Penicillium glaucum, Sclerophoma pityophila, Trichoderma viride.

### Beispiel E

### Wirkung gegen Bakterien

Ein Agar, der als Nährmedium Bouillon enthält, wird mit erfindungsgemäßen Wirkstoffen in Konzentrationen von 1 bis 5000 ppm versetzt. Darauf infiziert man das Nährmedium jeweils mit den in Tabelle II aufgeführten Testorganismen und hält das infizierte Medium 2 Wochen bei 28°C und 60 bis 70 % rel. Luftfeuchtigkeit. Die MHK ist die niedrigste Konzentration an Wirkstoff, bei der keinerlei Bewuchs durch die verwendete Mikrobenart erfolgt. Die MHK-Werte sind in Tabelle II wiedergegeben.

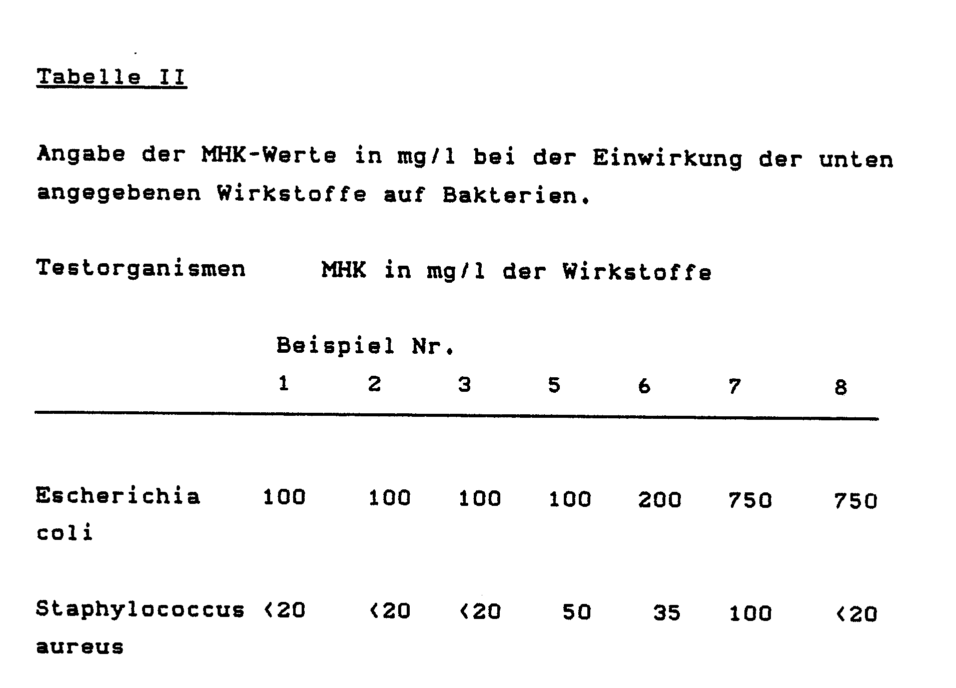

## Patentansprüche

1. N-Sulfenylierte 2-Trifluormethylbenzimidazole der allgemeinen Formel (I) 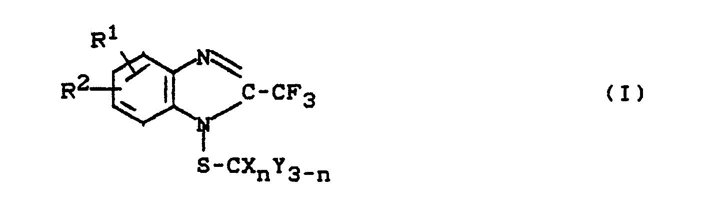 in welcher
X und Y unabhängig voneinander für Halogen stehen,
n für 0, 1, 2 oder 3 steht und
R¹ und R² gleich oder verschieden sind und für Wasserstoff, Halogen, jeweils gegebenenfalls durch Halogen substituiertes Alkyl, Alkoxy oder Alkylthio, für Nitro oder Alkylsulfonyl stehen oder gemeinsam für einen gegebenenfalls durch Halogen substituierten und gegebenenfalls durch Heteroatome unterbrochenen ankondensierten Ring stehen.

2. N-Sulfenylierte 2-Trifluormethylbenzimidazole der Formel (I) gemäß Anspruch 1, in welcher
X und Y unabhängig voneinander für Fluor, Chlor und Brom stehen,
n für 0, 1, 2 oder 3 steht und
R¹ und R² gleich oder verschieden sind und für Wasserstoff, Fluor, Chlor, Brom, jeweils gegebenenfalls ein- oder mehrfach durch Fluor, Chlor und/oder Brom substituiertes Alkyl mit 1 bis 6 Kohlenstoffatomen, Alkoxy und Alkylthio mit jeweils 1 bis 4 Kohlenstoffatomen, für Nitro oder Alkylsulfonyl mit 1 bis 4 Kohlenstoffatomen stehen oder R¹ und R² gemeinsam für einen 5-oder 6-gliedrigen, ankondensierten Ring stehen, welcher durch Sauerstoff, Stickstoff und/oder Schwefel unterbrochen und ein- oder mehrfach durch Fluor, Chlor und/oder Brom substituiert sein kann.

3. N-Sulfenylierte 2-Trifluormethyl-benzimidazole der Formel (I) gemäß Anspruch 1, in welcher
X und Y unabhängig voneinander für Fluor, Chlor und Brom stehen,
n für 2 oder 3 steht und
R¹ und R² gleich oder verschieden sind und für Wasserstoff, Fluor, Chlor, Brom, jeweils gegebenenfalls ein- bis fünffach durch Fluor, Chlor und/oder Brom substituiertes Alkyl mit 1 bis 4 Kohlenstoffatomen, Alkoxy oder Alkylthio mit jeweils 1 bis 3 Kohlenstoffatomen, für Nitro oder Alkylsulfonyl mit 1 oder 2 Kohlenstoffatomen stehen oder R¹ und R² gemeinsam für einen 5- oder 6-gliedrigen, ankondensierten Ring stehen, welcher durch 1 oder 2 Sauerstoff-und/oder Schwefelatome unterbrochen und ein-bis vierfach durch Fluor, Chlor und/oder Brom substituiert sein kann.

4. Verfahren zur Herstellung von N-Sulfenylierten 2-Trifluormethyl-benzimidazolen der Formel (I), 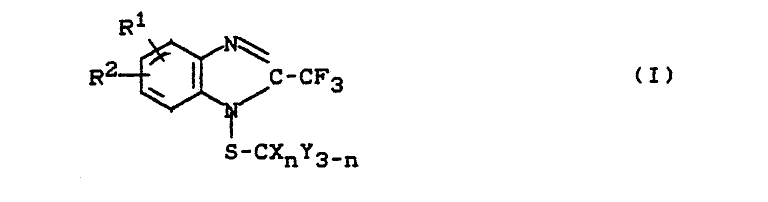 in welcher
X und Y unabhängig voneinander für Halogen stehen,
n für 0, 1, 2 oder 3 steht und
R¹ und R² gleich oder verschieden sind und für Wasserstoff, Halogen, jeweils gegebenenfalls durch Halogen substituiertes Alkyl, Alkoxy oder Alkylthio, für Nitro oder Alkylsulfonyl stehen oder gemeinsam für einen gegebenenfalls durch Halogen substituierten und gegebenenfalls durch Heteroatome unterbrochenen ankondensierten Ring stehen,
dadurch gekennzeichnet, daß man 2-Trifluormethylbenzimidazole der Formel (II) 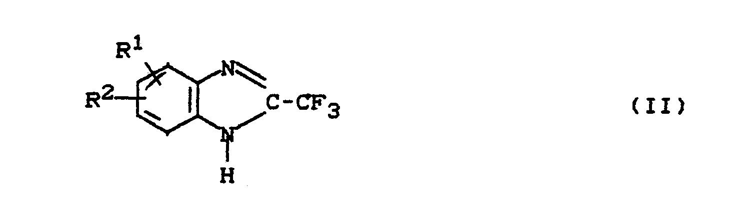
in welcher
R¹ und R² gleich oder verschieden sind und die oben angegebene Bedeutung haben,
mit Sulfensäurehalogeniden der Formel (III)
Z-S-CXₙY₃₋ₙ (III)
in welcher
X, Y und n die oben angegebene Bedeutung haben und
Z für Halogen, bevorzugt für Chlor oder Brom, steht,
in Gegenwart eines Verdünnungsmittels und in Gegenwart eines Säurebindemittels umsetzt.

5. Herbizide und mikrobizide Mittel, gekennzeichnet durch einen Gehalt an mindestens einem N-sulfenylierten 2-Trifluormethyl-benzimidazol der Formel (I) gemäß den Ansprüchen 1 bis 4.

6. Verfahren zur Bekämpfung von Unkräutern, dadurch gekennzeichnet, daß man N-sulfenylierte 2-Trifluor methyl-benzimidazole der Formel (I) gemäß den Ansprüchen 1 bis 4 auf die Unkräuter und/oder ihren Lebensraum einwirken läßt.

7. Verfahren zur Bekämpfung von Mikroben, dadurch gekennzeichnet, daß man N-sulfenylierte 2-Trifluormethyl-benzimidazole der Formel (I) gemäß den Ansprüchen 1 bis 4 auf Mikroben und/oder ihren Lebensraum einwirken läßt.

8. Verwendung von N-sulfenylierten 2-Trifluormethyl-benzimidazolen der Formel (I) gemäß den Ansprüchen 1 bis 4 zur Bekämpfung von Unkräutern.

9. Verfahren zur Herstellung von herbiziden und mikrobiziden Mitteln, dadurch gekennzeichnet, daß man N-sulfenylierte 2-Trifluormethyl-benzimidazole der Formel (I) gemäß den Ansprüchen 1 bis 4 mit Streckmitteln und/oder oberflächenaktiven Substanzen vermischt.
